# EUROPEAN PATENT APPLICATION

(11) **EP 2 386 246 A1**
(43) Date of publication of application: **16.11.2011**
(21) Application number: 10183619.5
(22) Date of filing: 06.05.2004
(51) Int. Cl.: A61B 5/06

(54) **Apparatus and method for navigating a catheter**

(30) Priority: 21.05.2003 EP 03101456
(62) Divisional of application: 04731441.4
(71) Applicant: Koninklijke Philips Electronics N.V., 5621 BA Eindhoven (NL); Philips Intellectual Property & Standards GmbH, 20099 Hamburg (DE)
(72) Inventor: Krüger, Sascha, 52066 Aachen (DE); Borgert, Jörn, 52066 Aachen (DE); Timinger, Holger, 52066 Aachen (DE)
(74) Representative: Gipp, Thomas

(57) **Abstract**

The invention relates to a navigation system for guiding a catheter in a patient's vascular system, where the spatial position of the catheter and its orientation are continually measured by a locating device. The resulting trajectory (To) of the catheter contains movement artefacts on account of the heartbeat. In order to suppress said movement artefacts, the electrocardiogram (ECG) is recorded in parallel, and the position and orientation signals are suppressed during phases of strong heart movement (QRS peak). Preferably, extrapolation of the compensated trajectory (T_{c}) is carried out in the gaps arising as a result of signal suppression.

## Description

The invention relates to a navigation system and a method for navigating a catheter in a vascular system which is subject to a cyclic intrinsic movement.

During a catheter examination carried out for diagnostic or therapeutic purposes, it is extremely important for the treating physician to know the current position of the instrument (catheter tip, guidewire, etc.) in the patient's vascular system in as precise a manner as possible. In clinical practice, this aim is usually achieved in that the movement of the catheter in the patient's body is subject to X-ray monitoring, wherein possibly radio-opaque markers are applied to the catheter. In order to have on the X-ray images also a best possible representation of the course of the vessel, an X-ray contrast agent is moreover injected from time to time. However, this procedure has the disadvantage of being a relatively high strain on the patient on account of the X-ray radiation and the contrast agent and - in terms of the X-ray radiation - also on the medical staff.

For the reasons given above, a catheter navigation is desired in which only a few X-ray images of the vascular system have to be taken with administration of a contrast agent and the catheter is then monitored on these static images or "road maps". The current spatial position and orientation of the catheter must in this case be determined by suitable methods such as, for example, location by means of a magnetic field. However, such a procedure using static road maps runs into problems when the monitored body volume moves and therefore no longer geometrically coincides with the static road maps. An overall movement of the patient may in this connection be practically ruled out as a cause of error by measures such as careful instruction, steady positioning and sedation of the patient. However, cyclic intrinsic movements of the organs of the body by breathing and heartbeat cannot be avoided. These movements are obviously particularly disruptive during examinations of organs in the chest area, such as the coronary vessels.

In this respect, DE 199 46 948 Al discloses a method which tries to achieve better position accuracy by using a number of road maps which are taken at different phases of a cyclic intrinsic movement of the body. In this case, an image database comprising a number of three-dimensional images of a periodically moving organ of the body, such as the heart for example, is generated prior to a catheter examination, with a movement signal (ECG, breathing signal) being recorded at the same time as the images. During the subsequent medical intervention, the spatial position of the instrument and also of a reference probe are determined by means of a locating device and the movement signal is recorded at the same time. By means of the movement signal, the relevant 3D image in terms of the movement phase of the organ of the body can then be selected from the image database and used for display purposes. One disadvantage of the known method is in particular the high outlay associated therewith.

Against this background, it is an object of the present invention to provide means for the simpler navigation of an instrument in a vascular system, with compensation of cyclic intrinsic movements of the body.

This object is achieved by a navigation system having the features of claim 1 and by a method having the features of claim 10. Advantageous refinements are contained in the dependent claims.

The navigation system according to the invention is used to navigate a catheter in a vascular system which is subject to a cyclic intrinsic movement caused by the heartbeat. In this case, the term "catheter" is to be understood in a broad sense and hereinafter encompasses any instrument which is to be moved in a controlled manner through the vascular system of a patient. The navigation system comprises the following components:
a) A locating device which is designed to record a temporal sequence of position signals, with each of the position signals indicating the spatial position of the catheter at the associated measurement time. In this connection, the term "position of the catheter" is to be understood to mean the spatial position *r*, expressed for example by Cartesian coordinates, of at least one selected point of the catheter, with the term "position" possibly also encompassing the orientation of the catheter expressed for example by three angles. Furthermore, the term "temporal sequence" refers to the fact that for each position signal the associated measurement time *t* is known as a temporal coordinate. The sequence therefore typically consists of spatial-temporal coordinates (*r, t*).
b) A measuring system for recording an electrocardiogram. The measuring system typically contains at least two electrodes by means of which the excitation potential of the heart can be derived. The electrodes may be attached externally to the body of the patient or be integrated in the catheter.
c) A data processing device which is coupled to the locating device and the measuring system and is designed to generate a compensated trajectory from the sequence of position signals, the compensated trajectory being obtained from the sequence by changing position signals that have been recorded during a predefined phase of the electrocardiogram. The change in a position signal may in particular consist in removing the latter or in replacing the latter by one or more replacement signals. Depending on the technical implementation of the navigation, a position signal can be "removed" by the obtaining thereof being suppressed during the predefined ECG phase. The predefined phase of the electrocardiogram is typically defined such that it corresponds to a strong spatial movement of the heart. In particular, the predefined phase of the ECG may therefore correspond to the systoles of the heartbeat.

By changing position signals during phases of a strong movement of the heart, the navigation system can generate a compensated trajectory which describes the position of the catheter without the disruptive influence of a heart movement. The compensated trajectory can therefore be used in particular to show the position of the catheter on a static road map which has been recorded during a heart phase outside the predefined signal change phases. The calculation of an informative compensated trajectory is therefore particularly possible because the spatial movement of the heart is essentially limited to phases of the heart cycle that can be clearly determined. Typically, a change of position signals must therefore take place approximately during 70% of the duration of a trajectory, whereas the trajectory can be used unchanged the rest of the time.

In the simplest case, the position signals are removed without being replaced during the predefined phases of the electrocardiogram. The compensated trajectory then contains gaps during which there are no position signals. Preferably, these gaps in the compensated trajectory are filled by interpolating and/or extrapolating retained position signals. An interpolation can be used if the compensated trajectory contains retained position signals before and after, in time terms, a gap that is to be filled. In the simplest case, the gap can in this case be closed by a linear interpolation between the last position signal before the gap and the first position signal after the gap. In many applications, this will primarily involve displaying, in real time, a currently measured trajectory for the treating physician. If the trajectory is therefore in a phase of the heart cycle in which its position signals are suppressed, no interpolation is possible since the continuation of the trajectory after the end of the suppression phase is (as yet) unknown. Thus, in order to be able to provide the treating physician with a current representation of the (approximate) catheter position, in these cases an extrapolation of the recorded trajectory in the gap will be carried out. This means that the profile of the trajectory in the gap is predicted on the basis of previous trajectory values.

A Kalman filter is preferably used for the abovementioned extrapolation of the trajectory. The Kalman filter allows the ideal prediction of the trajectory on the basis of a model on which the filter is based, the parameters of which are continuously adapted to the movement at any given time. It can be calculated relatively simply at the same time.

As mentioned above, the heartbeat and breathing are the most important cyclic intrinsic movements of the body which make navigation of a catheter more difficult. The trajectory generated by the navigation system, which has been compensated with respect to the heartbeat, thus usually still contains disruptions on account of breathing. The data processing device is therefore preferably also designed to correct the compensated trajectory with respect to an intrinsic movement of the vascular system caused by breathing.

In this respect, in the event of an optional type of breathing correction the data processing device is designed to carry out the following steps:
a) Calculation of a movement pattern caused by breathing from part-sections of the compensated trajectory in which there has been no movement (no advance or retreat) of the catheter relative to the vascular system. If required, the compensated trajectory is characterized in that the movement parts caused by the heartbeat are already eliminated. In one part-section - that is to say a group of temporally successive position signals - of the compensated trajectory in which it is known that there has been no relative movement of the catheter in the vascular system, any location change in the compensated trajectory must therefore be caused by the breathing movement. Such part-sections are therefore suitable for recognizing the movement pattern present at the associated location of the vascular system and caused by breathing. The movement pattern can in this case be described for example by periodically time-dependent difference vectors which on the basis of a spatially constant reference point allocate each point in time to a predefined point of the vascular system on the compensated trajectory. Part-sections of the compensated trajectory in which there is no relative movement of the catheter are preferably detected by an additional device in the navigation system, which additional device records for example an advance or a retreat of the catheter in the vascular system and provides this information in addition to the recorded sequence of the position signals.
b) Correction of the compensated trajectory by subtracting the movement pattern calculated in step a).

According to one variant for correcting the breathing movement, the data processing device is designed to correct the compensated trajectory by applying a spatial extrapolation filter (e.g. Kalman filter) on the basis of a previously determined movement pattern.

According to another development of the navigation system, the latter may comprise a breathing sensor that is coupled to the data processing device. A breathing sensor supplies a signal which represents a characteristic point in time of the breathing cycle and/or the phase profile of the breathing cycle. With the aid of this signal, the breathing correction of the trajectory can be compared and thus made even more precise, or alternative methods for correcting breathing movements may be used.

The invention furthermore relates to a method of navigating a catheter in a vascular system which is subject to a cyclic intrinsic movement caused by the heartbeat. The method comprises the following steps:
a) Recording of a temporal sequence of position signals which indicate the respective spatial position (position and possibly orientation) of the catheter.
b) Recording of an electrocardiogram in parallel, in time terms, with the sequence of position signals according to step a).
c) Determination of a predefined phase of the electrocardiogram. This may be in particular the phase of a systole which corresponds to a relatively strong movement of the heart.
d) Generation of a compensated trajectory from the temporal sequence of position signals recorded in step a), by changing in this sequence those position signals which have been recorded during the predefined phase of the electrocardiogram determined in step c).

The method comprises in a general manner the steps that can be carried out by the navigation system described above. For details regarding the refinement, advantages and developments of the method, reference should therefore be made to the explanations regarding the navigation system.

The compensated trajectory is continued during the predefined phases of the electrocardiogram preferably by means of an interpolation or extrapolation. In this way, the gaps resulting from the removal of position signals can be at least more or less closed, so that a continually updated position of the catheter is available to the treating physician.

The invention will be further described with reference to examples of embodiments shown in the drawings to which, however, the invention is not restricted.
Fig. 1 schematically shows the use of a navigation system according to the invention in a catheter examination of coronary vessels.
Fig. 2 shows a spatial representation of a measured catheter trajectory To and of a trajectory T_{c} that has been compensated with respect to the heartbeat, and also as an inset the parallel profile of the electrocardiogram.
Fig. 3 shows the profile of a typical electrocardiogram with the phases in which position signals are to be suppressed.

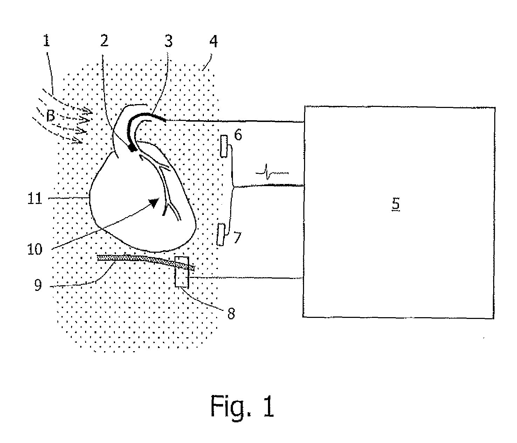

Fig. 1 schematically shows the components of a navigation system according to the invention, by means of which a catheter 3 can be guided in the vascular system 10 of a patient in order for example to examine the coronary vessels of the heart 11. At the tip of the catheter 3 there is a magnetic field probe 2 which can be used to measure the strength and direction of a magnetic field 1 impressed on the space by a field generator (not shown). The resulting measured signal is forwarded to a data processing device 5 (computer), where information about the current absolute spatial position of the probe 2 and hence of the catheter 3 can be obtained from the measured signals. The probe 2 is thus a locating device which supplies a temporal sequence of position signals relating to the current position r(t) = (x(t), y(t), z(t)) and orientation φ(t) = (α(t), β(t), γ(t)) (α = yaw angle, β = angle of inclination, γ = roll angle, t = time) of the catheter 3. Instead of the position determination with the aid of a magnetic field 1 which is shown by way of example, other methods could of course also be used to determine the current position and possibly orientation of the catheter 3.

The navigation system furthermore comprises electrodes 6, 7 for recording an electrocardiogram and also a breathing sensor 8 which monitors for example the movement of the diaphragm 9. The signals from these sensors are likewise passed to the data processing device 5.

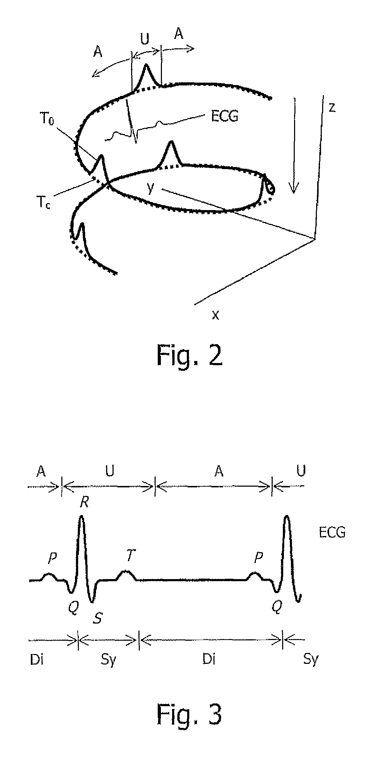

In order to minimize the exposure of the patient to X-ray radiation and contrast agent injections, it is endeavored that the movement of the catheter 3 be monitored on a few static X-ray images of the vascular system 10, known as "road maps". In this case, however, the intrinsic movement of the vascular system 10 in the thorax cavity 4 caused by the heartbeat and by breathing must be taken into account and compensated. In this respect, Fig. 2 shows the profile of a trajectory To in a spatial coordinate system with the axes x, *y* and *z*, said trajectory being formed from the sequence of position signals *r*(*t*) supplied by the magnetic sensor 2. The trajectory To shows, in the center, a movement running in the direction of the arrow which corresponds to the advance of the catheter along the vessel, this advance being performed by the physician. However, deflections caused by the heartbeat are superposed on this movement (these being shown in the figure as transverse deflections relative to the vessel).

For part of the trajectory To, Fig. 2 shows in parallel the electrocardiogram ("ECG") recorded at the same time. Deflections of the catheter which are caused not as a result of an advance of the catheter along the vessel but rather by the heartbeat are essentially restricted to the systolic phases of the heartbeat.

Fig. 3 shows an electrocardiogram in greater detail. The characteristic points of the ECG are denoted by the letters P, Q, R, S and T in the conventional manner. The systole Sy of the heart corresponds to the RST region, whereas the diastole Di encompasses the points P and Q. From empirical investigations, the ECG phases can be correlated with the resulting heart movement (cf. Y. Wang, E. Vidan, G.W. Bergman: "Cardiac Motion of Coronary Arteries: Variability in the Rest Period and Implications for Coronary MR Angiography", Radiology, 213:751-758, 1999; S. Achenbach, D. Ropers, J. Holle, et al.: "In-Plane Coronary Arterial Motion Velocity: Measurement with Electron Beam CT", Radiology, 216:457-463, 2000). In this way, it is possible to determine a phase U of the ECG during which data of the trajectory To of Fig. 2 are to be removed or suppressed since they contain movement artefacts on account of a strong movement of the heart. As shown in Fig. 3, this phase U of signal suppression contains in particular points Q, R and S of the electrocardiogram. On the other hand, during the remaining phases, denoted "A", the position signals of the trajectory To are retained unchanged. To analyze the profile of the electrocardiogram, use may be made for example of the methods described in the literature (cf. B.-U. Köhler, C. Hennig, R. Orglmeister: "The Principles of Software QRS Detection", IEEE Engineering in Medicine and Biology, pages 42-57 (2002)).

In the event of the trajectory generated by signal suppression in the phases U of the ECG being displayed directly in real time on a static road map, the catheter position appears frozen during a heart contraction (phase U) since no current position signals are available. As a result, there may be a certain discrepancy between the actual catheter position and the catheter position displayed for example on a monitor. In order to minimize this discrepancy, the profile of the trajectory is preferably extrapolated in the signal suppression gaps. Such an extrapolation may take place for example by means of a Kalman filter (R.E. Kalman, "A New Approach to Linear Filtering and Prediction Problems", Transactions of the ASME - Journal of Basic Engineering, 82 (Series D), 35-45, 1960; P.S. Maybeck: "Stochastic models, estimation, and control, Vol. I", Academic Press, 1979). The compensated trajectory obtained by signal suppression in the phases U and subsequent extrapolation is shown in dashed line in Fig. 2 and denoted T_{c}.

According to the invention the following exemplary embodiments are also provided:
1. A navigation system for navigating a catheter (3) in a vascular system (10) which is subject to a cyclic intrinsic movement caused by the heartbeat, comprising
   a) a locating device (2) for recording a temporal sequence (To) of position signals which indicate the respective spatial position of the catheter (3);
   b) a measuring system (6, 7) for recording an electrocardiogram (ECG);
   c) a data processing device (5) which is coupled to the locating device (2) and the measuring system (6, 7) and is designed to generate a compensated trajectory (T_{c}) from the sequence (To) by changing position signals that have been recorded during a predefined phase (U) of the electrocardiogram (ECG).
2. A navigation system according to embodiment 1, wherein the predefined phase (U) of the electrocardiogram (E) corresponds to the systole (Sy) of the heartbeat.
3. A navigation system according to embodiment 1, wherein the data processing device (5) is designed to carry out the change in the position signals by interpolation and/or extrapolation.
4. A navigation system according to embodiment 3, wherein the extrapolation takes place with the aid of a Kalman filter.
5. A navigation system according to embodiment 1, wherein in the data processing device (5) is designed to correct the compensated trajectory (T_{c}) with respect to an intrinsic movement of the vascular system (10) that is caused by breathing.
6. A navigation system according to embodiment 5, wherein the data processing device (5) is designed to carry out the following steps:
   a) calculation of a movement pattern caused by breathing from part-sections of the compensated trajectory (T_{c}) in which there has been no movement of the catheter (3) relative to the vascular system (10);
   b) correction of the compensated trajectory (T_{c}) by subtracting the calculated movement pattern.
7. A navigation system according to embodiment 5, wherein the data processing device (5) is designed to correct the compensated trajectory (T_{c}) by applying an extrapolation filter on the basis of a previously determined movement pattern.
8. A navigation system according to embodiment 1, wherein it has a breathing sensor (8) that is coupled to the data processing device (5).
9. A method of navigating a catheter (3) in a vascular system (10) which is subject to a cyclic intrinsic movement caused by the heartbeat, comprising the steps:
   a) recording of a temporal sequence (To) of position signals which indicate the respective spatial position of the catheter (3);
   b) recording of an electrocardiogram (ECG) in parallel with the sequence of position signals;
   c) determination of a predefined phase (U) of the electrocardiogram;
   d) generation of a compensated trajectory (T_{c}) by changing, in the sequence (To) of position signals, position signals which have been recorded during the predefined phase (U) of the electrocardiogram.
10. A method according to embodiment 9, wherein the compensated trajectory (T_{c}) is continued during the predefined phase (U) of the electrocardiogram (ECG) by means of interpolation and/or extrapolation.

## Claims

1. A navigation system for navigating a catheter (3) in a vascular system (10) which is subject to a cyclic intrinsic movement caused by the heartbeat, comprising
a) a locating device (2) for recording a temporal sequence (To) of position signals, which indicate the respective spatial position of the catheter (3);
b) a measuring system (6,7) for recording an electrocardiogram (ECG);
c) a data processing device (5),
coupled to the locating device (2) and the measuring system (6,7);
adapted to determine a predefined phase (U) of the electrocardiogram (ECG) during which predefined phase (U) data of the sequence (To) is removed or suppressed;
adapted to remove or suppress of said data of the sequence (T₀); and
adapted to generate a compensated trajectory (T_{c}) from the sequence (To) by interpolation and/or extrapolation of position signals, which have been removed or suppressed during the predefined phase (U).

2. A navigation system as claimed in the preceding claim, **characterized in that** the compensated trajectory (T_{c}) comprises the position of the locating device (2) on a static road map, which static road map has been recorded during a phase of the electrocardiogram outside the predefined phase (U) of the electrocardiogram (ECG).

3. A navigation system as claimed in the preceding claim, **characterized in that** suppression of data of the sequence (To) comprises suppression of data acquisition.

4. A navigation system as claimed in the preceding claim, **characterized in that** the predefined phase (U) of the electrocardiogram (E) corresponds to the systole (Sy) of the heartbeat.

5. A navigation system as claimed in at least one of the preceding claims, **characterized in that** the extrapolation takes place with the aid of a Kalman filter.

6. A navigation system as claimed in at least one of the preceding claims, **characterized in that** the data processing device (5) is designed to correct the compensated trajectory (T_{c}) with respect to an intrinsic movement of the vascular system (10) that is caused by breathing.

7. A navigation system as claimed in claim 6, **characterized in that** the data processing device (5) is adapted to carry out the following steps:
a) calculation of a movement pattern caused by breathing from part-sections of the compensated trajectory (T_{c}) in which there has been no movement of the catheter (3) relative to the vascular system (10);
b) correction of the compensated trajectory (T_{c}) by subtracting the calculated movement pattern.

8. A navigation system as claimed in claim 6 or 7, **characterized in that** the data processing device (5) is designed to correct the compensated trajectory (T_{c}) by applying an extrapolation filter on the basis of a previously determined movement pattern.

9. A navigation system as claimed in at least one of the preceding claims, **characterized in that** it has a breathing sensor (8) that is coupled to the data processing device (5).

10. A navigation system as claimed in at least one of the preceding claims, **characterized in that** the locating device (2) is a magnetic field probe.
